Europäisches Patentamt

European Patent Office

Office européen des brevets

(11) Publication number: 0 288 716 B1

# EUROPEAN PATENT SPECIFICATION

(45) Date of publication of patent specification: 02.09.92   (51) Int. Cl.⁵: **A61M 5/14**

(21) Application number: **88104196.6**

(22) Date of filing: **08.02.85**

(60) Publication number of the earlier application in accordance with Art.76 EPC: **0 154 191**

(54) **Cassette for an infusion system.**

<div style="columns:2">

(30) Priority: **08.02.84 US 578180**

(43) Date of publication of application:
**02.11.88 Bulletin 88/44**

(45) Publication of the grant of the patent:
**02.09.92 Bulletin 92/36**

(84) Designated Contracting States:
**BE CH DE FR GB IT LI LU NL SE**

(56) References cited:
**EP-A- 0 013 334     US-A- 4 142 523**
**US-A- 4 204 538     US-A- 4 236 880**
**US-A- 4 276 004     US-A- 4 303 376**

(73) Proprietor: **OMNI-Flow, Inc.**
**4e Henshaw Street**
**Woburn Massachusetts 01801(US)**

(72) Inventor: **Epstein,Paul**
**95 Cilnton Road**
**Brookline,Massachusetts 02146(US)**
Inventor: **Petschek,Harry**
**1314 Massachusetts Avenue**
**Lexington,Massachusetts 02173(US)**
Inventor: **La White,Eric**
**Dairy Hill**

**South Royalton,Vermont 05068(US)**
Inventor: **Strohl,Clair**
**3 Winston Road**
**Norfolk,Massachusetts 02056(US)**
Inventor: **Coyne,Henry**
**1622 Worcester Road**
**Framingham,Massachusetts 01701(US)**
Inventor: **Kaleskas,Edward**
**1662 Wachusett Street**
**Jefferson,Massachusetts 01522(US)**
Inventor: **Adaniya,George**
**120 Galloupe's Point Road**
**Swampscott,Massachusetts 01907(US)**

(74) Representative: **EGLI-EUROPEAN PATENT ATTORNEYS**
**Widenmayerstrasse 5**
**W-8000 München 22(DE)**

</div>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a cassette for an infusion system that administers selected infusates into the circulatory system of a patient according to the generic portion of claim 1.

US-A-4 303 376 discloses an infusion device for infusing a single fluid from a single fluid source. More particularly, this publication is directed toward a flow metering cassette for providing a single predetermined volume of infusate. This cassette comprises an actuable fluid input valve and an actuable output valve.

Other disposable cassettes or pump chambers for intravenous feeding systems or infusion systems are known from US-A-4 236 880, US-A-4 204 538 and from CA-A-1 110 137.

US-A-4 303 376 discloses only a disposable pump chamber with no internal valves. A valve action is provided merely by externally pinching off flow within the pump chamber and not by an externally operating and actuable valve.

CA-A-1 110 137 is related to a disposable pump assembly in the form of a cassette consisting basically of three plastic parts which form the pump body. The first is a bottom part, the second is a middle part and the third is a top part. A pump and valve membrane are provided between the middle and top parts.

These prior art cassettes are only suitable for infusion systems having a single fluid input port and a single output port.

In recent time, infusion systems having a single pump, several input ports and an output port have been developed. An infusion system of such type is described in EP-A-0013334. It comprises a single pump connected to the system output line and several valves in individual supply lines arranged between respective infusion bottles and the pump. The flow from each supply line is combined in the system output line. The system furthter comprises a computer for selectvely controlling in a cyclic sequence the respective actuation times of the valves and the respective pumping rate of the pump during the actuation of the respective valve.

In view of this prior art, the present invention is based on the object of providing a cassette for an infusion system which is suitable for an infusion system that administers selected infusates into the circulatory system of a patient and which has a design suitable for a large-scale series production.

This object is achieved by a cassette in accordance with features comprised by the characterizing portion of claim 1.

Hereinafter, a preferred embodiment of the cassette in accordance with the present invention will be described with reference to the attached drawings, wherein:

Fig. 1A
is a plan view illustrating one portion of a cassette of the infusion system having plural fluid input ports and at least one patient output port according to the present invention;

Fig. 1B
is a plan view illustrating another portion of the cassette of the infusion system having plural fluid input ports and at least one patient output port according to the present invention;

Fig. 1C
is a plan view illustrating a flexible diaphragm of the cassette of the infusion system having plural fluid input ports and at least one patient output port according to the present invention; and

Fig. 1D and 1E
are sectional views of the cassette taken along the lines D-D and E-E of Figs. 1A-1C of the infusion system having plural fluid inport ports and at least one patient output port according to the present invention.

The cassette in accordance with the present invention can be applied to an infusion system as disclosed in the European Patent Application 85 101 328.4.

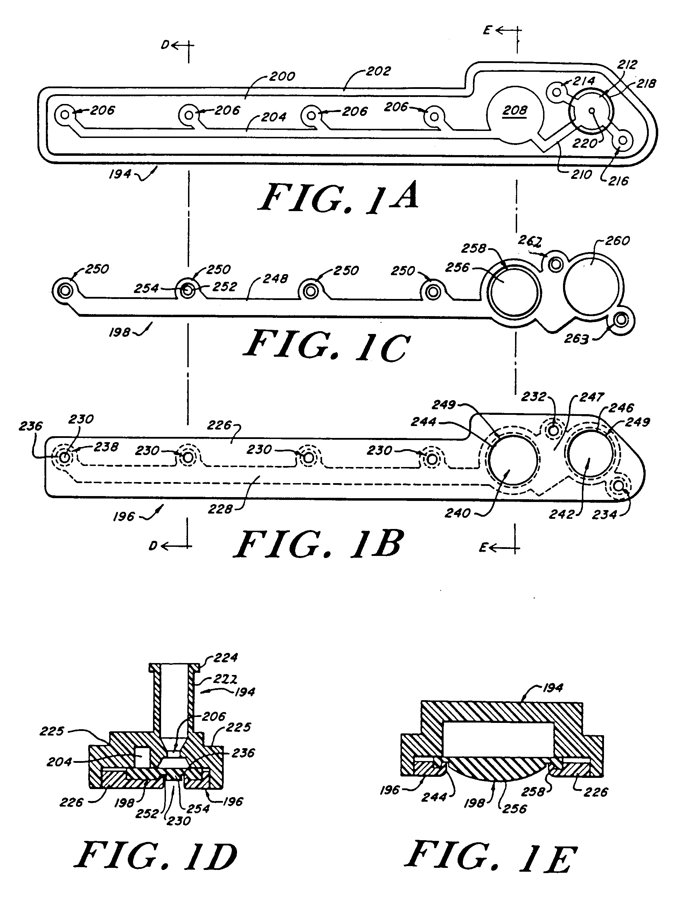

Generally illustrated at 194 in Fig. 1A is a first housing portion, generally designated at 196 in Fig. 1B is a second housing portion, and generally designated at 198 in Fig. 1C is a flexible diaphragm of a disposable cassette of the infusion system having plural fluid input ports and at least one patient output port according to the present invention. As shown in Fig. 1A, the housing portion 194 includes an injection molded clear plastic member 200 that meets appropriate U.S. Pharmacopia standards. The member 200 includes an integral upstanding peripheral flange 202 and a longitudinally extending fluid flow channel 204. A plurality of longitudinally spaced fluid input apertures generally designated 206 and a pumping chamber generally designated 208 are integrally formed with the member 200 in communication with the fluid flow path channel 204. A channel 210 is integrally formed with the plastic material 200 between the pumping chamber 208 and a pressure chamber generally designated 212. The chamber 212 is integrally formed with the plastic material 200. A patient output aperture generally designated 214 and a vent output aperture generally designated 216 are integrally formed with the plastic material 200 and are in fluid communication with the pressure chamber 212. A disc 218 having a central aperture 220 is provided over the pressure chamber 212 that cooperates with the walls defining the pressure chamber to prevent the collapse of the diaphragm 198 (Fig. 1C) into the chamber 212. As best seen in Fig. 1D, the cassette housing portion 194 includes an annulus 222 defining an input fluid port integrally formed surrounding a corresponding one of the fluid apertures 206, 214, 216 (Fig. 1A). Diametrically opposed locking flanges 224 are integrally formed on the ends of each annulus 222. The plastic member 200 includes longitudinally extending shoulders 225 that abut longitudinally extending guides provided therefor on the side of the housing that prevents the movement of the cassette in a direction transverse to its plane.

Referring now to Fig. 1B, the housing portion 196 includes a clear plastic member 226 that mates in fluid tight sealing engagement with the housing portion 194 (Fig. 1A). The member 226 includes a longitudinally extending diaphragm receiving recess 228. A plurality of longitudinally spaced input valve plunger receiving apertures generally designated 230 are provided through the plastic member 226. An output valve plunger receiving aperture 232 is provided in the plastic member 226 and a vent valve plunger receiving aperture 234 is provided in the plastic member 226. An upstanding annular flange 236 integrally formed with the plastic member 226 is provided surrounding each of the input valve plunger receiving apertures 230, the vent valve plunger receiving aperture 234, and the output valve plunger receiving aperture 232. A semicircular channel portion generally designated 238 integrally formed in the plastic member 226 is provided surrounding each of the annular flanges 236 that are in communication with the channel 228. The plastic member 226 of the housing portion 196 includes a pumping piston receiving aperture generally designated 240 and a pressure transducer receiving aperture generally designated 242. An annular flange 244 integrally formed in the plastic member 226 in communication with the channel 228 is provided surrounding the aperture 240, and an annular flange 246 integrally formed in the plastic member 226 is provided surrounding the aperture 242. Semicircular channel portions generally designated 249 are also provided around the annular flanges 244, 246. A recess 247 is provided intermediate the flanges 244, 246 forming a continuation of recess 228. The ends of the flanges 236, 244, 246 are flush with the generally planar surface of the plastic member 226.

Referring now to Fig. 1C, the diaphragm 198 is preferably an injection molded length of silicone rubber that meets the appropriate U.S. Pharmacopia standards. The diaphragm 198 includes a longitudinally extending reinforced seal portion 248 having a transverse width greater than the transverse width of the longitudinally extending fluid channel 204 (Fig. 1A) that is received in the recess 228 (Fig. 1B). A plurality of longitudinally spaced input fluid valve pads generally designated 250 are provided on the longitudinally extending reinforced seal portion 248. Individual ones of the valve pads 250 are aligned with corresponding ones of the apertures 206 (Fig. 1A) and apertures 230 (Fig. 1B). The valve pads 250 include an annular recess 252 that is individually aligned with a corresponding one of the annular flanges and an integral upstanding cylindrical projection 254 that are individually aligned with corresponding ones of the apertures 206 (Fig. 1A) and apertures 238 (Fig. 1B).

A convex dome 256 surrounded by an annular recess generally designated 258 is provided on the diaphragm 198. The recess 258 is aligned with the annular flange 244 (Fig. 1B) and the dome 256 is aligned with the aperture 240 (Fig. 1B) and the pumping chamber 208 (Fig. 1A). A thin circular portion 260 is provided on the diaphragm 198. The portion 260 is aligned with the flange 246 (Fig. 1B) and with the pressure chamber 218 (Fig. 1A). A vent valve pad generally designated 262 is provided on the diaphragm 198 between the members 256, 260 in alignment with the apertures 216 (Fig. 1A) 234 (Fig. 1B), and a patient output valve pad generally designated 263 is provided adjacent the cylindrical depression 258 in alignment with the apertures 214 (Fig. 1A), 232 (Fig. 1B). Each of the pads 262, 263 include an integral upstanding cylindrical projection surrounded by an annular recess like those described above for the pads 250. The cylindrical projections of the valve pads 250, 262, 263 have dimensions larger from the dimensions of the corresponding aligned apertures of the member 194 to provide a seal thereagainst to prevent fluid flow. The thickness of the portions 248, 256 (Fig. 1C) is selected to provide a stiffness sufficient to prevent their unintended collapse into the portions 204, 208 (Fig. 1A) during operation.

In the assembled condition of the disposable cassette as best seen in Figs. 1D and 1E, the diaphragm 198 is sandwiched between the housing portion 194 and the housing portion 196. The longitudinally extending seal portion 248 of the diaphragm 198 is received in the diaphragm receiving recess 228, the solid cylindrical projections 254 of the valve pads 250, 262, 263 extend into corresponding ones of the apertures 230, 232, 234, the dome portion 256 is received over the mouth of the pumping chamber 208, and cylindrical depression is received over the disc 218 and pressure chamber 212. Any suitable means such as ultrasonic welding may be employed to secure the two housing portions together in fluid tight sealing engagement. The cassette is oriented in use preferably at 45° to the vertical. As will readily be appreciated, any air in the fluid flow channel 204 Fig. 1A) rises upwardly therealong through the pumping chamber 208 (Fig. 1A) and fluid path 210 into the pressure chamber 212 (Fig. 1A). As appears below, the system controller is operative to detect any air in the pressure chamber and to appropriately open the vent output valve to vent the air and to alarm should the condition persist. Since the air rises upwardly into the pressure chamber, the pumping chamber in normal operation is substantially free of air. When the pumping chamber is controllably exhausted, only the intended infusate is administered into the patient output port thereby preventing the possibility of admitting air into the patient.

Individual ones of a plurality of valve plungers are received in corresponding ones of the apertures 230, 232, 234 (Fig. 1B) that are reciprocally moveable to push corresponding upstanding cylindrical projections 254 (Fig. 1D) into sealing contact with the apertures 206, 214, 216 to control the state of actuation of the corresponding fluid valves. The cyclindrical projections with their associated plunger withdrawn flex out of contact with the corresponding apertures to allow fluid flow into and out of the pumping chamber 208. A pumping piston to be described is received in the pumping piston receiving aperture 240 (Fig. 1B). The piston is reciprocally moveable to controllably push the dome 256 (Fig. 1C) into the pumping chamber 208 as can best be seen in Fig. 1E. The fluid that accumulates therein during each pumping sequence to be described is thereby pumped through the patient output port and into the circulatory system of a patient. The rate of reciprocating motion of the pumping piston, its travel distance into the chamber 208, and the time interval between pumping strokes is selected to controllably administer intended volumes of infusant in intended time intervals.

## Claims

1. Cassette for an infusion system that administers selected infusates into the circulatory system of a patient, comprising:
a cassette housing (194, 196) including a longitudinally extending fluid flow channel (204) being in fluid communication with at least one fluid input port (206) and with at least one patient output port (214),
selectively actuable valve means for both the at least one fluid input port (206) and the at least one patient output port (214),
means (240, 256, 258) for providing a pumping chamber (208) in fluid communication with the at least one fluid input port (206) and in fluid communication with the at least one patient output port (214) through the respective associated valve means,
characterized in that

means (236) are formed with the cassette housing (194, 196) and in fluid communication with the fluid flow channel (204) for providing a plurality of fluid input ports (206) spaced apart longitudinally along the fluid flow channel (204), each input port having means (222) for receiving an input line from a selected infusate source,
means (250, 254) are incorporated in the cassette and coupled to each of the fluid input ports (206) for providing a plurality of selectively actuable valves in the cassette in the corresponding fluid input ports (206),
the means (240, 256, 258) for providing the pumping chamber (208) are formed with the cassette housing (194, 196), which pumping chamber (208) is in fluid connection with the fluid input ports (206) through the corresponding valve.

2. Cassette as claimed in claim 1, characterized by means (242, 260) in fluid communication with the fluid flow channel (204) for providing a pressure chamber (212) for monitoring by an external pressure transducer.

3. Cassette as claimed in claim 1, characterized by means (214, 232) in fluid communication with the fluid flow channel (204) for providing a vent port (216), and means (232) coupled to the vent port (216) for providing a valve in the vent port (216).

4. Cassette as claimed in one of the claims 1 to 3, characterized in that said pumping chamber (208) is externally actuable by a pumping piston.

5. Cassette as claimed in one of the claims 1 to 4, characterized in that said cassette is transparent.

## Patentansprüche

1. Kassette für ein Infusionssystem, das ausgewählte Infusionslösungen dem Kreislaufsystem eines Patienten verabreicht, mit:
einem Kassettengehäuse (194, 196), das einen sich in Längsrichtung erstreckenden Durchflußkanal (204) aufweist, der in Strömungsverbindung mit mindestens einem Flüssigkeitseinlaß (206) und mindestens einem zum Patienten führenden Auslaß (214) steht,
selektiv betätigbaren Ventileinrichtungen für den mindestens einen Flüssigkeitseinlaß (206) und den mindestens einen zum Patienten führenden Auslaß (214),
Mitteln (240, 256, 258), die eine Pumpkammer (208) bilden, welche in Strömungsverbindung mit dem mindestens einen Flüssigkeitseinlaß (206) und über entsprechend zugeordnete Ventileinrichtungen mit dem mindestens einen zum Patienten führenden Auslaß (214) steht, dadurch gekennzeichnet,

daß Mittel (236) im Kassettengehäuse (194, 196) in Strömungsverbindung mit dem Durchflußkanal (204) vorgesehen sind, die eine Vielzahl von Flüssigkeitseinlässen (206) bilden, die in Längsrichtung längs des Durchflußkanales (204) im Abstand zueinander angeordnet sind, wobei jeder Einlaß Mittel (222) aufweist zum Anschließen einer Zufuhrleitung von einer ausgewählten Infusionslösungsquelle,

daß Mittel (250, 254) in der Kassette beinhaltet und an jeden der Flüssigkeitseinlässe (206) gekoppelt sind, die eine Vielzahl von selektiv betätigbaren Ventilen der Kassette in den entsprechenden Flüssigkeitseinlässen (206) bilden,

daß die Mittel (240, 256, 258) zur Bildung der Pumpkammer (208) in dem Kassettengehäuse (194, 196) ausgebildet sind, wobei die Pumpkammer (208) über die entsprechenden Ventile in Strömungsverbindung mit den Flüssigkeitseinlässen (206) steht.

**2.** Kassette nach Anspruch 1, gekennzeichnet durch Einrichtungen (242, 260), die in Strömungsverbindung mit dem Durchflußkanal (204) stehen und eine Druckkammer (212) zur Überwachung durch einen externen Druckwandler bilden.

**3.** Kassette nach Anspruch 1, gekennzeichnet durch Einrichtungen (214, 232), die in Strömungsverbindung mit dem Durchflußkanal (204) stehen und die eine Entlüftungsöffnung (216) bilden sowie durch Mittel (232), die an die Entlüftungsöffnung (216) gekoppelt sind und ein Ventil in der Entlüftungsöffnung (216) bilden.

**4.** Kassette nach einem der Ansprüche 1 bis 3, dadurch gekennzeichnet, daß die Pumpkammer (208) extern durch einen Pumpenkolben betätigbar ist.

**5.** Kassette nach einem der Ansprüche 1 bis 4, dadurch gekennzeichnet, daß die Kassette transparent ist.

**Revendications**

**1.** Cassette pour un système de perfusion qui administre des fluides de perfusion sélectionnés dans le système circulatoire d'un patient comprenant:
- un boîtier de cassette (194, 196) comportant un canal d'écoulement de fluide (204) qui s'étend dans le sens longitudinal et est en communication de fluide avec au moins un orifice d'entrée de fluide (206) et avec au moins un orifice de sortie de fluide (214) vers un patient,
- des moyens de valve, susceptibles d'être actionnés sélectivement, pour ledit orifice d'entrée de fluide (206) et ledit orifice de sortie de fluide (214) vers un patient,
- des moyens (240, 256, 258) définissant une chambre de pompage (208) en communication de fluide avec ledit orifice d'entrée de fluide (206) et en communication de fluide avec ledit orifice de sortie (214) vers un patient, à travers les moyens de valve respectifs associés, caractérisée en ce que:
- des moyens (236) sont formés avec le boîtier de cassette (194, 196) et en communication de fluide avec le canal (204) d'écoulement de fluide, pour réaliser une pluralité d'orifices d'entrée de fluide (206) espacés longitudinalement le long du canal (204) d'écoulement de fluide, chaque orifice d'entrée comportant des moyens (224, 222) aptes à recevoir une ligne d'entrée en provenance d'une source sélectionnée de fluide de perfusion,
- des moyens (250, 254) sont incorporés dans la cassette et couplés à chacun des orifices d'entrée de fluide (206) pour réaliser une pluralité de valves susceptibles d'être actionnées sélectivement, dans la cassette, aux orifices d'entrée de fluide (206) correspondants, les moyens (240, 256, 258) pour réaliser la chambre de pompage (208) sont formés dans le boîtier (194, 196) de la cassette, cette chambre de pompage (208) étant en liaison de fluide avec les orifices d'entrée de fluide (206) à travers la valve correspondante.

**2.** Cassette selon la revendication 1, caractérisée par des moyens (242, 260) en communication de fluide avec le canal d'écoulement de fluide (204) pour réaliser une chambre de pression (212) permettant une surveillance par un capteur externe de pression.

**3.** Cassette selon la revendication 1, caractérisée par des moyens (214, 232) en communication de fluide avec le canal d'écoulement de fluide (204) pour réaliser un orifice de purge (216), et des moyens (232) couplés à l'orifice de purge (216) pour réaliser une valve dans l'orifice de purge (216).

4. Cassette selon l'une des revendications 1 à 3, caractérisée en ce que ladite chambre de pompage (208) est susceptible d'être actionner de l'extérieur par un piston de pompage.

5. Cassette selon l'une des revendications 1 à 4, caractérisée en ce que ladite cassette est transparente.

FIG. 1A

FIG. 1C

FIG. 1B

FIG. 1D

FIG. 1E